# EUROPEAN PATENT SPECIFICATION

(11) **EP 2 321 312 B1**
(45) Date of publication and mention of the grant of the patent: **23.12.2015**
(21) Application number: 09788631.1
(22) Date of filing: 30.07.2009
(51) Int. Cl.: C07D 471/04

(54) **PROCESS FOR THE PREPARATION OF PALIPERIDONE**
VERFAHREN ZUR HERSTELLUNG VON PALIPERIDON
PROCÉDÉ DE PRÉPARATION DE LA PALIPÉRIDONE

(30) Priority: 31.07.2008 SI 200800190
(43) Date of publication of application: 18.05.2011
(73) Proprietor: KRKA, d.d., Novo mesto, 8501 Novo mesto (SI)
(72) Inventor: RUZIC, Milos, 3000 Celje (SI); PRUDIC, Darja, 8000 Novo mesto (SI); PECAVAR, Anica, 8000 Novo mesto (SI); SMRKOLJ, Matej, 1411 Izlake Zagorje ob Savi (SI)
(74) Representative: Redensek, Vladimira
(86) International application number: PCT/SI2009/000032
(87) International publication number: WO 2010/014047

(56) References cited:
- EP-A- 0 368 388
- EP-A- 1 281 420
- WO-A-2008/015005
- WO-A-2008/021345
- WO-A-2009/056990
- US-A- 5 158 952
- ROGERS ROBIN D ET AL: "Chemistry. Ionic liquids--solvents of the future?" SCIENCE (NEW YORK, N.Y.) 31 OCT 2003, vol. 302, no. 5646, 31 October 2003 (2003-10-31), pages 792-793, XP002553258 ISSN: 1095-9203

## Description

### Technical Field

The present invention belongs to the field of chemical synthesis and relates to an improved process for the preparation of paliperidone and its pharmaceutically acceptable salts as an active ingredient of a medicament for the treatment of schizophrenia.

### Prior Art

Paliperidone with the chemical name 3-[2-[4-(6-fluoro-1,2-benzisoxazol-3-yl)-1-piperidinyl]ethyl]-6,7,8,9-tetrahydro-9-hydroxy-2-methyl-4Hpyrido[1,2-a]pyrimidin-4-one is a racemic mixture with the following structural formula:

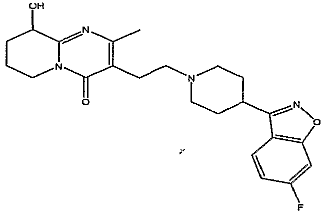

Paliperidone is an active metabolite of risperidone and belongs to the second generation of antipsychotics, also named atypic antipsychotics. It acts as a dual antagonist of dopamine D2 receptors and serotonine 5-HT_{2A} receptors and is used for the treatment of schizophrenia.

In EP 0 368 388 there is described a multi-step synthesis of paliperidone comprising a nucleophilic substitution between 3-(2-chloroethyl)-6,7,8,9-tetrahydro-9-hydroxy-4H-pyrido[1,2-a]pyrimidin-4-one and 6-fluoro-3-(4-piperidinyl)-1,2-benzisoxazole hydrochloride in the presence of methanol and diethylamine and recrystallization from 2-propanol. Disadvantages of the mentioned process are low yields and purification of the product with column chromatography.

In WO 2008/021345 there is described a synthesis of paliperidone from intermediates 3-(2-chloroethyl)-6,7,8,9-tetrahydro-9-hydroxy-4H-pyrido[1,2-a]pyrimidin-4-one and 6-fluoro-3-(4-piperidinyl)-1,2-benzisoxazole in the presence of an inorganic base. Yields are somehow improved in view of EP 0 368 388, however in the process organic solvents are used which are known as harmful due to two simple reasons, they are easily volatile and on an industrial scale they are used in large quantities.

WO 2008/021342 describes amorphous and six polymorphic forms of paliperidone. IPCOM000167996D describes recrystallization of paliperidone from 2-propanol or ethanol.

In addition to the above-mentioned processes for the preparation of paliperidone, pharmaceutical industry has a constant need for the improved preparation of paliperidone.

Thus, the subject of the present invention is to provide a process for the production of paliperidone, which would be cost-, time- and economically-effective, technologically simple and suitable for industrial scale and by which high yields and high quality of the final product would be achieved.

### Figures

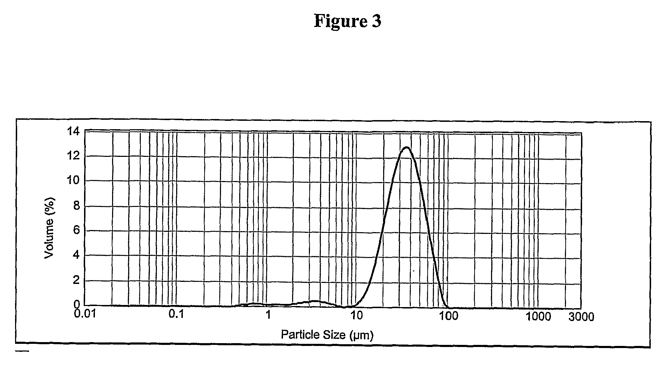

Figure 3 represents histogram of size distribution of paliperidone particles prepared according to Example 10. Size distribution of paliperidone particles was determined by laser diffraction by the use of laser diffraction instrument Malvern Mastersizer 2000. Analysis samples were prepared by dispersing the weighed amount of paliperidone particles in Izopar L.

### Summary of the Invention

The subject of the present invention is to provide a process for the preparation of paliperidone comprising a use of dispergator with stirring rates from 8000 to 24000 rounds/minute in a crystallization phase, which enables obtaining uniform and smaller basic particles of paliperidone with narrow size distribution.

### Detailed Description of the Invention

The subject of the present invention is to provide a process for the preparation of paliperidone comprising a use of dispergator with stirring rates from 8000 to 24000 rounds/minute in a crystallization phase, which enables obtaining of uniform and smaller basic particles of paliperidone with narrow size distribution. Further advantages are repeatability, easier control of the course of crystallization and possibility of direct use of the obtained paliperidone particles in the process of preparation of a pharmaceutical composition, and besides, more uniform particles enable constant dissolving. As in a crystallization phase dispergator with stirring rates from 8000 to 24000 rounds/minute is used, no additional milling is necessary.

The dispergator with stirring rates from 8000 to 24000 rounds/minute can be selected from numerous commercially available products used in laboratory and/or industry, such as Ultra-Turrax^{®}.

Suitable solvents for crystallization are methanol, ethanol, propanol, isopropanol, butanol, isobutanol, tert.-butanol, acetonitrile, THF, ethyl acetate and their mixtures which may optionally also contain water.

The obtained paliperidone particles may have an average diameter between 3-100 µm, preferably between 5-70 µm and d₉₀ between 7-100 µm, preferably between 10-70 µm. Diameter of particles is controlled by adjusting of process parameters of the dispergator with stirring rates from 8000 to 24000 rounds/minute. Diameter of particles is inversely proportional to number of rounds/minute.

If in the crystallization phase no dispergator with stirring rates from 8000 to 24000 rounds/minute is used, as a rule, particles of an average diameter of 5-250 µm are obtained. Without use of the dispergator, it is much more difficult to control their average diameter with parameters such as cooling rate, stirring mode and stirring rate and similar.

Stirring rates with dispergator, which can be used, include, but are not limited to, from 8000 rounds/minute to 24000 rounds/minute.

The present invention is illustrated, but in no way limited by the following Example.

### Examples

### Example 10

### Process of crystallization of paliperidone

5 g of paliperidone were suspended in 300 ml of 2-propanol, heated to boiling temperature of the mixture, hot filetered and begun with cooling. At 57 °C stirring of a suspension (the product already nucleated) started for a short time (5-10 minutes) with dispergator Ultra-Turrax® at 8000 rounds/minute. It was cooled to room temperature, filtered, sucked off. The mass of the moist product was 4.1 g. We put it on an analysis wherein an average diameter was 37 µm, D₉₀ was 60 µm.

| | |
|---|---|
| Average diameter | 37 µm |
| d10 | 17.8 µm |
| d50 | 34.1 µm |
| d90 | 59.9 µm |

## Claims

1. A process of crystallization of paliperidone, **characterized in that** in a crystallization phase a dispergator with stirring rates from 8000 to 24000 rounds/minute is used.

2. A process of crystallization of paliperidone according to claim 1, **characterized in that** as a crystallization solvent methanol, ethanol, propanol, isopropanol, butanol, isobutanol, tert.-butanol, acetonitrile, THF, ethyl acetate and their mixtures which optionally may also contain water are used.

## Patentansprüche

1. Verfahren zur Kristallisation von Paliperidon, **dadurch gekennzeichnet, dass** in einer Kristallisationsphase ein Dispergator mit Rührraten von 8000 bis 24.000 Umdrehungen/Minute verwendet wird.

2. Verfahren zur Kristallisation von Paliperidon nach Anspruch 1, **dadurch gekennzeichnet, dass** als Kristallisationslösungsmittel Methanol, Ethanol, Propanol, Isopropanol, Butanol, Isobutanol, tert.-Butanol, Acetonitril, THF, Essigsäureethylester und Mischungen davon, die gegebenenfalls auch Wasser enthalten können, verwendet werden.

## Revendications

1. Procédé de cristallisation de palipéridone, **caractérisé en ce que**, dans une phase de cristallisation, un dispergateur avec des vitesses d'agitation de 800C à 24000 tours/minute est utilisé.

2. Procédé de cristallisation de palipéridone selon la revendication 1, **caractérisé en ce que**, en tant que solvant de cristallisation, le méthanol, l'éthanol, le propanol, l'isopropanol, le butanol, l'isobutanol, le tert-butanol, l'acétonitrile, le THF, l'acétate d'éthyle et leurs mélanges qui peuvent facultativement contenir de l'eau sont utilisés.
